# EUROPEAN PATENT APPLICATION

(11) **EP 4 435 725 A1**
(43) Date of publication of application: **25.09.2024**
(21) Application number: 23163230.8
(22) Date of filing: 21.03.2023
(51) Int. Cl.: G06T 11/00

(54) **REAL-TIME INSTRUMENT DELINEATION IN ROBOTIC SURGERY**

(71) Applicant: Orsi Academy bv, 9090 Melle (BE)
(72) Inventor: De Backer, Pieter, 9090 Melle (BE); Mottrie, Alexandre, 9090 Melle (BE)
(74) Representative: Brantsandpatents bv

(57) **Abstract**

The current invention relates to a method for real-time artificial object delineation in robotically assisted surgery, as well as a system for performing said method.

## Description

### Fl ELD OF THE I NVENTI ON

The present invention relates to a systems and methods for providing augmented reality based assistance. In particular, the present invention relates to methods and systems for providing augmented reality based assistance in a medical environment.

### BACKGROUND

US 2011/282140 A1 discloses a method and system of hand segmentation and overlay using depth data. The method includes starts with the acquisition a plurality of video images, each image including a hand pose image. Depth data for the hand pose image is also acquired or synthesized. The hand pose image is segmented from the image using the depth data. The segmented image is combined with an acquired surgical site image using the depth data. The combined image is displayed to a person at a surgeon's console of the minimally invasive surgical system. Processing each of the video images in the plurality video images in this way reproduces the hand gesture overlaid on the video of the surgical site in the display.

US 2022/101533 A1 discloses a method and system for combining computer vision techniques to improve segmentation and classification of a surgical site. Edges of areas of interest within a surgical site are detected using a combination of image segmentation techniques based on color, brightness or other differences in the image data captured of the surgical site. Edges of the areas of interest are also determined through an analysis of depth data derived from stereoscopic images or other 3D images of surgical site. The image data is displayed with overlays marking both edges as detected using the image segmentation techniques and edges as determined from the depth data.

US 2011/026794 A1 discloses a method for performing deformable non-rigid registration of 2D and 3D images of a vascular structure for assistance in surgical intervention. The method includes acquiring 3D image data. An abdominal aorta is segmented from the 3D image data using graph-cut based segmentation to produce a segmentation mask. Centerlines are generated from the segmentation mask using a sequential topological thinning process. 3D graphs are generated from the centerlines. 2D image data is acquired. The 2D image data is segmented to produce a distance map. An energy function is defined based on the 3D graphs and the distance map. The energy function is minimized to perform non-rigid registration between the 3D image data and the 2D image data.

The methods and systems cited above focus only on a single aspect of medical procedure information, making them unsuitable to provide a medical practitioner with sufficient real-time awareness of the medical procedure. The present invention aims to provide a method and system with solves these shortcomings.

### SUMMARY OF THE I NVENTI ON

The present invention and embodiments thereof serve to provide a solution to one or more of above-mentioned disadvantages. To this end, the present invention relates to a method for providing a real-time augmented-reality image of a medical procedure, preferably a surgical procedure, according to claim 1.

Preferred embodiments of the method are shown in any of the claims 2 to 11.

In a second aspect, the present invention relates to a system for robot-assisted medical, preferably surgical, operations supported by real-time augmented reality support, according to claim 12.

Preferred embodiments of the system are shown in any of the claims 13 to 15.

### DESCRIPTI ON OF FIGURES

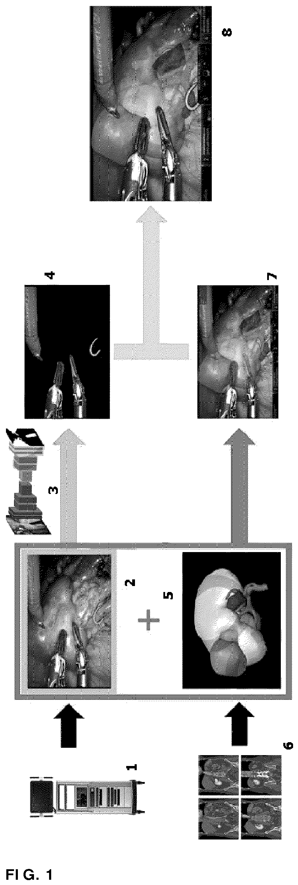

**Figure 1** shows a schematic overview of a method according to an embodiment of the invention.

### DETAILED DESCRIPTION OF THE INVENTION

The present invention concerns a method for providing a real-time augmented-reality image of a medical procedure. The method provides a medical professional with augmented reality image containing information of the medical procedure and/or a body part combined with full visibility of each non-organic object present in the image during the medical procedure. This full visibility solves all occlusion problems, removing all doubt about the location of all surgical equipment and body parts. In this way, the safety of a surgical procedure being carried out is guaranteed. The visibility allows the medical professional to, for example, avoid any inadvertent contact between surgical instruments and blood vessels during a surgical procedure, which would otherwise cause the patient to lose blood and potentially bleed out. The visibility provided by the augmented reality images also permits enhanced anatomical comprehension and tool-tissue interaction when carrying out surgeries by intermediate of a medical robot.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g., component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.*, any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In a first aspect, the invention relates to a method for providing a real-time augmented-reality image of a medical procedure, the method comprising the steps of:
capturing an image of the medical procedure;
automatically identifying and segmenting non-organic objects in said captured image and segment the identified the non-organic objects in the captured image by a pretrained machine learning model;
creating a first mask comprising only the segmented non-organic objects from the captured images wherein only non-organic objects are represented, wherein said first mask is created by using a pretrained machine learning model to automatically identify said non-organic objects in said captured image and segment the identified non-organic objects in the captured images;
creating a second mask or overlay comprising medical information relating to the medical procedure and/or relating to one or more body parts visible in said captured image;
combining said captured image with said first mask and said second mask or overlay;

The first mask is applied over the second mask or overlay. This method advantageously solves occlusion problems and provide a medical professional with augmented reality image with full visibility of the medical procedure. This full visibility removes all doubt about the location of all surgical equipment and body parts, thereby increasing the safety of the surgical procedure being carried out. The visibility allows the medical professional to, for example, avoid any inadvertent contact between surgical instruments and blood vessels during a surgical procedure, which would otherwise cause the patient to lose blood and potentially bleed out. The visibility provided by the augmented reality images also permits enhanced anatomical comprehension and tool-tissue interaction when carrying out surgeries by intermediate of a medical robot.

In this context, the term non-organic objects is to be understood as any object and materials that are not part of the patient's body, even if said material or object is of animal or vegetable origin. In this way, all bodies and materials foreign to the body of a patient and introduced during surgery can be accounted for and located during a medical procedure. Most specifically in the invention, the term refers to medical instruments used during the procedure, covering such objects as surgical instruments (forceps, scissors, etc.), but also medical supplies (gauze, clamps, hooks, wires) and other objects.

In a preferred embodiment, the captured images are images in a captured video feed, and are subsequently processed and augmented according to the method of the invention. It is clear that, while applying this to single images is useful, the invention is particularly advantageous for video feeds, allowing near instantaneous display of augmented video feeds that provide full visibility of all non-organic/artificial elements in the field of view of the camera, as well as additional visual information on the feed.

In an embodiment, the machine learning model is trained to identify and segment at least one or more surgical instruments as non-organic objects. Surgical instruments are the non-organic objects that are most frequently handled by a medical practitioner and the most likely to cause damage to a patient's body if mishandled. By being able to identify and provide information related to the position of the surgical instruments, the safety of a medical intervention is greatly improved. By preference, the machine learning model is trained with models of a plurality of types of non-organic objects used in the medical procedure, said types comprising at least one or more of needles, gauze, wires, clamps, wires, trocars, forceps, scissors, catheter, drain, endograph elements, fibrillar elements, foam, clips, needle drivers, suction, hemostasis elements, vessel loops, gloves and patches.

In an embodiment, the captured images are preprocessed before automatically identifying and segmenting, said preprocessing comprising the preparatory steps of:
removing alpha-channel information from the captured images.

In this context, the term "alpha-channel" is to be understood as the channel of the captured images which carries pixel transparency information. Said channel is typically ignored by image classification algorithms, thus being unnecessary. By removing the alpha-channel, the volume of data to be processed during segmentation is advantageously reduced. This volume of data is further reduced by resizing the image to a smaller size. In this way, processing speed is advantageously increased, thereby reducing latency.

In an embodiment, the captured images are preprocessed before automatically identifying and segmenting, said preprocessing comprising the preparatory steps of:
performing normalization of color channels based on training set data of the machine learning model.

In an embodiment, the captured images are preprocessed before automatically identifying and segmenting, said preprocessing comprising the preparatory steps of:
resizing image to a smaller format.

In this context, the terms "normalization of color channels" are to be understood as the preprocessing of the color channels of the captured data in order to reduce color variability and improve the generalization of algorithms by transforming the input data to a common space. In this way, success of the segmentation of the non-organic objects in the captured image is advantageously guaranteed.

In an embodiment, the second mask comprises at least one 3D representation of a body part. By preference, said 3D representation is a dynamic representation, wherein the geometry of said 3D representation is adapted in real-time as response to body reactions/functions e.g. heart-beat, inhaling and exhaling. Most preferably, the geometry of said 3D representation is further adapted in real-time as a response interactions with any non-organic objects, e.g. body tissue deforms when clamped by a hemostatic clamp or when being cut. In this way, a medical practitioner is kept aware of any positional shifting of relevant body parts or elements thereof. This adaptation of the geometry of a 3D representation may be triggered by a signal from device for measuring a biological indicator, such as a heart-beat monitor, and/or by implementing an segmentation algorithm directed at identifying and segmenting a specific body part.

In an embodiment, the captured image is a stereoscopic image. In this way depth perception is very advantageously made possible. This allows a medical practitioner to maintain a sensorial capability that is essential to the execution of precise motions, therefore, to the safety and proper execution of the medical procedure. This is particularly advantageous when carrying out robot assisted surgical interventions.

In an embodiment, the masks are applied over the captured images with alpha-channel information. In this way, color information of the original images is advantageously maintained, permitting the presentation of augmented reality images of the medical procedure which are faithful to reality.

In a preferred embodiment, the second mask or overlay is an overlay and is applied on the captured image, optionally comprising a step of resizing the overlay before applying the resized overlay on the captured image.

The overlay aims to provide an augmented reality layer over the originally captured image. This can for instance comprise a theoretical model of some of the body parts in the image, or use previous images of the same patient to provide a more practical model, which is then shown along with the original image. Other or further information can for instance be sensor data, such as heart rates, temperature, etc., or even markers indicating certain objects or regions. Such markers can be smart and automated (for instance, automatically highlighting bleeding), but may also be manually placed by an observer (remote or present) via an interface. It is crucial that the second mask or overlay is an overlay and not a mask, meaning that it doesn't block out the underlying image, but combines with it, to provide a composite image. This way, both 'layers' are still visible to the observer, giving them the advantage of still seeing the actual body part(s) as well as seeing the info of the overlay. The overlay has a certain opacity associated to it, which can be determined by a user and changed, to present both options.

The creation of the second mask or overlay typically requires some further steps, depending on the type of information that is shown. In case of the superposition of (3D) models of an organ that is visible in the image, the model will need to be rotated and resized such that it correctly fits over the actual organ in the image, in order to provide relevant information for the user. Other such steps may be automatic adjustment of opacity, shade and/or other parameters, depending on the image conditions for the original image.

The overlay is preferably provided with a predetermined opacity, optionally dynamically variable, depending on the background, i.e., the originally captured image.

In some embodiments, the overlay comprises multiple separate overlays, which may or may not be toggleable. Each overlay may be provided with distinct characteristics, such as opacity.

The above does not preclude displaying certain information in a mask, and not in an overlay, for instance when the information is highly important and requires to be displayed very clearly. This can also be performed by providing an opacity value for such a piece of information that is 100% (or near such a value).

In a preferred embodiment, the step of applying of the first mask comprises substituting pixels of the combination of the captured image and the second mask or overlay, with the pixels from the captured image at the position of the to be substituted pixels, wherein said substitution is performed for pixels at whose position the machine learning model identified and segmented the non-organic objects.

The creation of the first mask is done via a segmentation of the processed image, resulting in one or more segments, where the segments represent regions of pixels that are identified as artificial objects, typically non-organic material. This first mask is then reapplied to the image, already augmented with the second mask or overlay, such that any pixel in image that is positioned in one of the segments representing artificial objects, are replaced by the pixel at said position of the mask, namely the pixel of the originally captured image at said position. This way, it is guaranteed that there is no overlay or second mask present at these positions, and that the artificial objects remain visible at all times, instead of being (partially) obscured by the overlay/second mask. By using the (pixels of the) originally captured image, it is further ensured that the resulting composite image (with first and second mask or overlay) is of the highest resolution possible.

In some embodiments, the captured images need further processing before they can be segmented (efficiently). This may include cropping, to remove any undesirable sections that could slow down the segmentation process, resizing and/or color normalization, the latter to improve the segmentation process by making objects more easily identifiable.

The trained machine learning model is released on the processed image, delineates artificial objects (as discussed earlier), and generates the first mask based on this segmentation.

It is clear that, when applying the masks and overlays, these typically can need to be resized and translated in order to align correctly with the image over which they are positioned (for instance, when the mas/overlay is based on a cropped or resized image).

In a second aspect, the invention relates to a system for robot-assisted medical, preferably surgical, operations supported by real-time augmented reality support, the system comprising:
a surgical robot comprising at least one arm equipped with at least one surgical instrument;
an image capturing device for capturing images of the area affected by said at least one surgical instrument;
an image processing unit comprising at least an image feed input for receiving the images captured by the imaging capturing device, a processing element for processing the received images, and a memory element;
and optionally a display unit for displaying the processed images from the image processing unit;

said memory element comprising:
   medical information relating to the medical procedure and/or relating to one or more body parts,
   a trained machine learning model;
   instructions for detecting and segmenting any non-organic objects from said captured images,
the processing unit further configured for:
   generating a first mask from the segmented non-organic objects
   generating a second mask or overlay comprising information relating to the medical procedure and/or relating to one or more body parts visible in the captured image, and
   overlaying the second mask or overlay on the captured image, and subsequently applying the first mask over the second mask or overlay and the captured image.

Robot assisted medical procedures deprive a medical practitioner of at last some relevant sensorial capabilities. In particular, said practitioner is no longer able to have a direct tactile feel of instruments, body parts of the patient which could be used to compensate for lack of visual access or clarity caused by, for example, blood or other body parts. The present system not only removes any shortcomings associated with a medical practitioner's lack of direct access to the body of the patient, it improves upon traditional medical procedures by providing an augmented reality image with full visibility of the medical procedure. This full visibility removes all doubt about the location of all surgical equipment and body parts, thereby increasing the safety of the surgical procedure being carried out. The visibility allows the medical professional to, for example, avoid any inadvertent contact between surgical instruments and blood vessels during a surgical procedure, which would otherwise cause the patient to lose blood and potentially bleed out.

In an embodiment, the image capturing device is an endoscope. Preferably the image capturing device is a stereoscopic endoscope. In order to take full advantage of this feature, the display unit includes a stereoscopic display. This allows the medical practitioner to have depth perception during the medical procedure. This greatly improve the control over the surgical instruments by the practitioner. The combination of the superior visibility offered by the system with input motion filtering, the system provides vastly superior control over the medical instruments and the medical procedure, greatly improving patient safety.

By preference, the display unit for displaying the masked video feed produced by the image processing unit and a control unit are integrated together. This permits improving the comfort of the medical practitioner operating the robot, thereby permitting long-duration medical procedures to be carried out.

Another advantage of the system is its capability as a teaching aid and as a means for post operatory control of a medical procedure.

However, it is obvious that the invention is not limited to this application. The method according to the invention can be applied in all sorts of procedures wherein a distinction between organic tissue and non-organic objects is necessary. The method can easily be used in veterinary or botanical procedures with little to no adaptation necessary. The method can also be used in machine maintenance, assembly and operation, chemical processes or any other processes where at least one object of known geometry must be moved relative to another object of known geometry.

The present invention will be now described in more details, referring to examples that are not limitative.

### EXAMPLES AND/OR DESCRIPTION OF FIGURES

With as a goal illustrating better the properties of the invention the following presents, as an example and limiting in no way other potential applications, a description of a preferred application of the method according to the invention. FIG. 1 presents a stepwise representation of the method according to the invention. With the aid of an imaging apparatus (1), a live image feed (2) is provided of a surgery in process, showing the body parts on which the physician is operating. These captured images are prepared for processing and subsequently processed by the machine learning model (3), resulting in a first mask (4) in which the artificial elements (surgical instruments, gauze, hooks, clamps, forcepts, etc.) that were identified and segmented by the machine learning model, are visualized. It is of particular note that holes, openings, etc., in these elements are marked as such, and do not form part of the segments.

In parallel, the captured images (2) are combined with an augmented reality overlay of a 3D model (5) of one of the organs visible in the feed. The model can be generated from earlier medical images (6) from the same patient, for maximal relevance. This 3D model is then rotated, resized and positioned over the image feed as an overlay with some transparency, thereby not entirely obfuscating the underlying original image, generating a combined image (7).

In the last step, the mask created by the machine learning model is applied, such that the segmented sections of the original image are reapplied over the combined image (7) with the augmented reality overlay created in the previous step. The application of the so-called instrument mask, is achieved by replacing pixels positioned in segmented sections, by the pixels of the original captured image at said positions, thus bypassing any augmented layer, resulting in a single composite image (8), with the augmented reality overlay, without blurring out or partially hiding instruments and other artificial elements in the images.

It is supposed that the present invention is not restricted to any form of realization described previously and that some modifications can be added to the presented example of fabrication without reappraisal of the appended claims. For example, the present invention has been described referring to surgical procedures, but it is clear that the invention can be applied to more general medical procedures as well as other types of remote procedures which can take place with robot assistance, for instance demining, repairs, manufacturing, etc., and in general, any type of procedures where robotically assisted tool handling can take place.

It is clear that the method according to the invention, and its applications, are not limited to the presented examples.

The present invention is in no way limited to the embodiments described in the examples and/or shown in the figures. On the contrary, methods according to the present invention may be realized in many different ways without departing from the scope of the invention.

## Claims

1. Method for providing a real-time augmented-reality image of a medical procedure, the method comprising the steps of:
capturing an image of the medical procedure;
automatically identifying and segmenting non-organic objects in said captured image and segment the identified the non-organic objects in the captured image by a pretrained machine learning model;
creating a first mask comprising only the segmented non-organic objects from the captured images wherein only non-organic objects are represented, wherein said first mask is created by using a pretrained machine learning model to automatically identify said non-organic objects in said captured image and segment the identified non-organic objects in the captured images;
creating a second mask or overlay comprising medical information relating to the medical procedure and/or relating to one or more body parts visible in said captured image;
combining said captured image with said first mask and said second mask or overlay;
**characterized in that** the first mask is applied over the second mask or overlay.

2. The method according to claim 1, **characterized in that**, the machine learning model is trained to identify and segment at least one or more surgical instruments as non-organic objects.

3. The method according to any of the preceding claims 1-2, **characterized in that**, the machine learning model is trained with models of a plurality of types of non-organic objects used in the medical procedure, said types comprising at least one or more of needles, gauze, wires, clamps, wires, trocars, forceps, scissors, catheter, drain, endograph elements, fibrillar, foam, clips, needle drivers, suction, hemostasis elements, vessel loops, gloves and patches.

4. The method according to any of the preceding claims 1 to 3, **characterized in that**, the captured images are preprocessed before automatically identifying and segmenting, said preprocessing comprising the preparatory step of:
removing alpha-channel information from the captured images.

5. The method according to any of the preceding claims 1 to 4, **characterized in that** the captured images are preprocessed before automatically identifying and segmenting, said preprocessing comprising the preparatory step of:
performing normalization of color channels based on training set data of the machine learning model.

6. The method according to any of the preceding claims 1 to 5, **characterized in that**, the second mask comprises at least one 3D representation of a body part.

7. The method according to any of the preceding claims 1 to 6, **characterized in that**, the captured image is a stereoscopic image.

8. The method according to any of the preceding claims 1 to 7, **characterized in that**, the masks are applied over the captured images with alpha-channel information.

9. The method according to any of the preceding claims 1 to 8, wherein the second mask or overlay is an overlay and is applied on the captured image, optionally comprising a step of resizing the overlay before applying the resized overlay on the captured image.

10. The method according to any of the preceding claims 1 to 9, wherein the step of applying of the first mask comprises substituting pixels of the combination of the captured image and the second mask or overlay, with the pixels from the captured image at the position of the to be substituted pixels, wherein said substitution is performed for pixels at whose position the machine learning model identified and segmented the non-organic objects.

11. The method according to any of the preceding claims 1 to 10, wherein the captured image is provided to a capture card of a processing unit as a digital signal, and converted into an encoded bitstream by said processing unit, wherein said processing unit performs the steps of creating the first mask, the second mask or overlay and combining the first mask, the second mask or overlay and the captured image

12. System for robot-assisted medical, preferably surgical, operations supported by real-time augmented reality support, the system comprising:
a. a surgical robot comprising at least one arm equipped with at least one surgical instrument;
b. an image capturing device for capturing images of the area affected by said at least one surgical instruments;
c. an image processing unit comprising at least an image feed input for receiving the images captured by the imaging capturing device, a processing element for processing the received images, and a memory element;
d. and optionally a display unit for displaying the processed images from the image processing unit, said display unit preferably comprising a stereoscopic display;
**characterized in that**,
said memory element comprises:
medical information relating to the medical procedure and/or relating to one or more body parts,
a trained machine learning model
instructions for detecting and segmenting any non-organic objects from said captured images,
the processing unit further configured for:
generating a first mask from the segmented non-organic objects
generating a second mask or overlay comprising information relating to the medical procedure and/or relating to one or more body parts visible in the captured image, and
overlaying the second mask or overlay on the captured image, and subsequently applying the first mask over the second mask or overlay and the captured image.

13. System according to the preceding claim 12, **characterized in that**, the image capturing device is an endoscope, preferably a stereoscopic endoscope.

14. System according to any of the preceding claims 12 to 13, **characterized in that**, the system comprises a display unit for displaying the masked video feed produced by the image processing unit and a control unit for controlling the robotic arm, said display unit and said control unit being integrated together.

15. System according to any of the preceding claims 12 to 14, wherein said system is configured for executing the method according to any of the preceding claims 1 to 11.
